(19) Europäisches Patentamt
European Patent Office
Office européen des brevets

(11) Veröffentlichungsnummer: **0 266 723**
**A1**

(12) # EUROPÄISCHE PATENTANMELDUNG

(21) Anmeldenummer: **87116086.7**

(22) Anmeldetag: **02.11.87**

(51) Int. Cl.⁴: **C07C 59/305** , C07C 51/00 , C11D 3/20

(30) Priorität: **05.11.86 DE 3637665**

(43) Veröffentlichungstag der Anmeldung:
**11.05.88 Patentblatt 88/19**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Reinhardt, Gerd, Dr.**
**Freiherr-vom-Stein-Strasse 37**
**D-6233 Kelkheim (Taunus)(DE)**
Erfinder: **Holst, Arno, Dr.**
**Drususstrasse 3**
**D-6200 Wiesbaden(DE)**
Erfinder: **Grabley, Fritz-Feo, Dr.**
**Hölderlinstrasse 7**
**D-6240 Königstein/Taunus(DE)**

(54) **Verfahren zur Herstellung von 1-Hydroxi-3-oxapentan-1,2,4,5-tetracarbonsäure und 3,6-Dioxaoctan-1,2,4,5,7,8-hexacarbonsäure, deren Salzen und deren Mischungen.**

㊼ Verfahren zur Herstellung von 1-Hydroxi-3-oxapentan-1,2,4,5-tetracarbonsäure und 3,6-Dioxa-octan-1,2,4,5,7,8-hexacarbonsäure, deren Salzen und deren Mischungen, wobei man ohne Zwischenisolierung a) 1 Mol Maleinsäureanhydrid mit 0.2 bis 1.5 Mol Wasserstoffperoxid in Gegenwart von Epoxidations-Katalysatoren epoxidiert, b) das dabei erhaltene Reaktionsgemisch durch Erhitzen hydrolysiert, c) nach Zugabe von Erdalkali- oder Zinksalzen als Katalysator bei pH Werten von 10 bis 14 auf 60 bis 110°C erhitzt und dann d) aus der so erhaltenen Lösung die oben genannten Endprodukte isoliert.

Bei diesem Eintopfverfahren entfällt der separate Einsatz von teurer Weinsäure und man erhält die genannten Endprodukte in einer vereinfachten Verfahrensweise.

EP 0 266 723 A1

Xerox Copy Centre

**Verfahren zur Herstellung von 1-Hydroxi-3-oxapentan-1,2,4,5-tetracarbonsäure und 3,6-Dioxaoctan-1,2,4,5,7,8-hexacarbonsäure, deren Salzen und deren Mischungen**

Die Erfindung betrifft ein Verfahren zur Herstellung der Etherpolycarbonsäuren 1-Hydroxi-3-oxapentan-1,2,4,5-tetracarbonsäure, im folgenden abgekürzt TMS (Tartratmonosuccinic acid), und 3,6-Dioxaoctan-1,2,4,5,7,8-hexacarbonsäure, im folgenden abgekürzt TDS (Tartratdisuccinic acid), derer Salze sowie derer Mischungen in einer mehrstufigen Reaktionsfolge ohne Zwischenisolierung ausgehend von Maleinsäuranhydrid oder Maleinsäure und Wasserstoffperoxid.

Etherpolycarbonsäuren sind seit langem bekannte Verbindungen, die unter anderem als Builder oder Phosphatersatz in Wasch-und Reinigungsmitteln Verwendung finden können. Sie besitzen ein hohes $Ca^{2+}$ und $Mg^{2+}$-Bindevermögen und sind meist biologisch gut abbaubar (Tenside 12, 47 (1975)). Bekannte Vertreter dieser Verbindungsklasse sind Oxidibernsteinsäure (ODS) (US 3,128,287) und Carboximethoxibernsteinsäure (CMOS), zu deren Herstellung und Isolierung eine Reihe von Verfahren bekannt sind (US 3,914,297, DE-OS 2 738 825, DE-OS 2 812 194). Die Herstellung erfolgt durch Addition einer hydroxylgruppenhaltigen Komponente an die Doppelbindung von Maleinsäure im Sinne einer Michael-Addition (Soap, Cosmetics, Chem. Spec. 1977, July S. 34). Eine analoge Addition von Weinsäure an ein Molekül Maleinsäure führt zu TMS, doppelte Addition zu TDS. Nachteilig bei diesem Verfahren ist jedoch die Verwendung von Weinsäure, da deren Herstellung, insbesondere deren Isolierung sehr kostenintensiv ist (DE-OS 2 016 668, DE-PS 2 140 055, DE-OS 2 543 333, DE-OS 2 555 699).

Aufgabe der vorliegenden Erfindung ist es, ein auch im großtechnischen Maßstab durchführbares Verfahren zur Herstellung von TMS/IDS aufzuzeigen, bei dem auf den Einsatz von Weinsäure verzichtet werden kann.

Es wurde nun gefunden, daß TMS/TDS in einer "Eintopfreaktion" ausgehend von einer wäßrigen Maleinsäure-Lösung und Wasserstoffperoxid hergestellt werden kann, ohne daß die als Zwischenstufe auftretende Weinsäure in aufwendiger Weise isoliert werden muß.

Gegenstand der Erfindung ist ein Verfahren zur Herstellung von 1-Hydroxi-3-oxapentan-1,2,4,5-tetracarbonsäure (TMS) der Formel

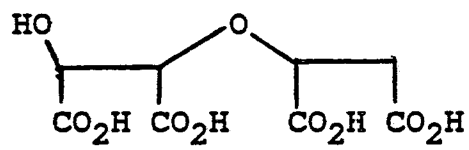

und

3,6-Dioxaoctan-1,2,4,5,7,8-hexacarbonsäure (TDS) der Formel

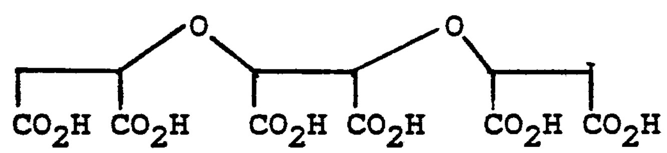

,

deren Salzen, insbesondere Alkali-und/oder Erdalkalimetallsalze, und deren Mischungen, wobei man ohne Zwischenisolierung

a) 1 Mol Maleinsäureanhydrid mit 0,2 bis 1,5 Mol Wasserstoffperoxid in Gegenwart von Epoxidationskatalysatoren epoxidiert,

b) das dabei erhaltene Reaktionsgemisch durch Erhitzen in wäßrigem Medium hydrolysiert,

c) nach Zugabe von Erdalkali-oder Zinksalzen als Katalysator bei pH Werten von 10 bis 14 auf 60 °C bis 110 °C erhitzt und dann

d) aus der so erhaltenen Lösung die oben genannten Endprodukte isoliert.

Im einzelnen läßt sich das Verfahren wie folgt beschreiben:

In Stufe a) wird eine wäßrige Lösung von Maleinsäure, vorzugsweise hergestellt durch Lösen von Maleinsäureanhydrid in Wasser, mit Wasserstoffperoxid partiell oder vollständig epoxidiert. Das verwendete Wasserstoffperoxid wird als wäßrige Lösung mit einem Gehalt von 20-80 %, vorzugsweise 30-35 %, 50 % oder 70 % eingesetzt. Das Molverhältnis Maleinsäure : Wasserstoffperoxid beträgt 1 : 0,2-1,5 vorzugsweise 1 : 0,45-0,7 oder 1 : 0,8-1,2. Als Katalysator für diese Epoxidation können die in J. Catal. 19, 256-263 (1970) beschriebenen Katalysatoren verwendet werden, insbesondere jedoch Salze des Wolframs oder

Molybdäns wie $Na_2WO_4$ oder $Na_2MoO_4$ oder Mischungen davon. Der Katalysator kann in wasserlöslicher Form oder auf polymerem Trägermaterial (Ionenaustauscher), wie z.B. in US 3 156 709 beschrieben, eingesetzt werden, wodurch seine Wiedergewinnung vereinfacht wird. Die Menge an Katalysator beträgt 0,1-10 Mol %, vorzugsweise 0,2-2 Mol %, bezogen auf eingesetzte Maleinsäure. Die Reaktionstemperatur beträgt 20-110 °C, vorzugsweise 50-90 °C, die Reaktionszeit 10 min - 10 h, vorzugsweise 30 min - 4 h. Die Reaktion wird bei pH-Werten kleiner 7 durchgeführt, insbesondere kleiner pH 3 oder pH-kontrolliert zwischen pH 4 und 6, was durch Einstellen mit einer anorganischen Base, z.B. Natriumhydroxid, erreicht werden kann. Desgleichen kann der pH-Wert auch während der Epoxidation korrigiert werden. An diese Epoxidationsstufe a), bei der schon ein Teil des gebildeten Epoxides zur Weinsäure hydrolysieren kann, - schließt sich die eigentliche Hydrolysestufe b) an. Dabei wird das in Stufe a) erhaltene Reaktionsgemisch - gegebenenfalls unter Zusatz von weiterem Maleinsäureanhydrid oder Maleinsäure - 10 min bis 10 h, vorzugsweise 30 min bis 3 h, in wäßriger Lösung auf 50-110 °C, vorzugsweise 80-105 °C, erhitzt. Die Reaktion kann auch unter Druck durchgeführt werden.

Bei dieser Reaktionsstufe können die in DE-OS 2 400 767 oder DE-PS 2 418 569 beschriebenen Hydrolysekatalysatoren wie Aktivkohle, $Al_2O_3$, $Fe_2O_3$, $AlCl_3 \bullet 6H_2O$ oder $FeCl_3 \bullet 6H_2O$ zur Beschleunigung der Reaktion zugesetzt werden. An diese Hydrolysestufe kann sich gegebenenfalls ein Filtrationsschritt anschließen, in dem der polymergebundene Epoxidationskatalysator und/oder der Hydrolysekatalysator abgetrennt werden kann. Wurde als Epoxidationskatalysator ein wasserlösliches Wolfram-oder Molybdänsalz eingesetzt, kann dieses mittels eines Ionenaustauschers entfernt werden. Die Abtrennung der Epoxidationskatalysatoren kann auch bereits nach der Reaktionsstufe a) erfolgen.

Zu dem nach Stufe b) vorliegenden Weinsäure-Maleinsäure-Gemisch kann weitere Weinsäure bzw. Maleinsäure zugegeben werden, um eine für die folgende Additionsreaktion (Stufe c) notwendige Stöchiometrie der Komponenten zu erhalten. Eine solche Zugabe von Maleinsäure ist beispielsweise dann nötig, wenn in der Stufe a) mit einem Molverhältnis Maleinsäure zu Wasserstoffperoxid von 1:0,8 bis 1,2 gearbeitet wurde, da in diesem Fall die ursprünglich vorhandene Maleinsäure weitgehend epoxidiert worden ist. Das Molverhältnis Weinsäure : Maleinsäure sollte 1 : 0,5-2,5, vorzugsweise 1: 0,8-1,2, betragen, falls TMS das Hauptprodukt der Reaktion sein soll; für die Herstellung von TDS beträgt das Molverhältnis vorzugsweise 1 : 1,8-2,1. Zu dem Maleinsäure/Weinsäuregemisch, das außerdem geringe Anteile von Fumarsäure - entstanden durch Isomerisierung von Maleinsäure - enthalten kann, wird Natriumhydroxid sowie ein Additionskatalysator gegeben. Die Menge an Natriumhydroxid, das in Form von Pulver oder als Lösung zugesetzt werden kann, richtet sich nach der Menge an Additions-Katalysator und ist so bemessen, daß der pH-Wert der Reaktionslösung nach Zugabe des Katalysators pH 9-14, vorzugsweise 10,5 bis 13, insbesondere 11,5-13,0, beträgt (gemessen als 10 %ige Lösung). Die Katalysatormenge beträgt 0,1-3,0 Mol, vorzugsweise 0,5-1,2 Mol, bezogen auf vorhandene Weinsäure. Als Katalysatoren können eingesetzt werden: Erdalkali-oder Zinksalze in Form ihrer Halogenide, Oxide, Carbonate, Hydrogencarbonate oder Hydroxide. Bevorzugt verwendet werden Calciumhydroxid und/oder Calciumcarbonat. Die Reaktionszeit der Additionsstufe ist temperaturabhängig und liegt zwischen 10 min und 16 h bei Reaktionstemperaturen zwischen 60 °C und 110 °C, bevorzugt werden jedoch kurze Reaktionszeiten von 1-5 h bei 80-105 °C. Zur Reaktionskontrolle eignet sich insbesondere die Hochdruckflüssigkeitschromatographie. Die Reaktion kann durch Verdünnung mit Wasser Erniedrigung des pH-Wertes oder Temperaturabsenkung unter 50 °C unterbrochen werden.

An die Additionsstufe c) schließt sich die Aufarbeitung-und Reinigungsstufe d) an, bei der das Reaktionsprodukt TMS/TDS von Nebenprodukten sowie Calciumionen befreit wird. Im Reaktionsgemisch vorhandene Weinsäure, insbesondere das D,L-Gemisch, kann durch Verdünnung mit Wasser und/oder Ansäuern der Reaktionslösung auf pH-Werte kleiner 9,5 bevorzugt auf pH 4-9, als schwerlösliches Calcium-DL-Tartrat gefällt werden. Zum Ansäuern des Reaktionsgemisches eignen sich anorganische oder organische Säuren wie Salzsäure, Schwefelsäure, Oxalsäure oder Essigsäure, bevorzugt werden jedoch D,L-Weinsäure oder Kohlensäure verwendet, um das Gemisch nicht durch weitere Komponenten zu verunreinigen. Der Niederschlag besteht zum überwiegenden Teil aus Calcium-Tartrat, jedoch können unterschiedliche Mengen an Produkt (TMS/TDS) sowie Maleinsäure oder Fumarsäure eingeschlossen sein. Das abfiltrierte Calcium-Tartrat kann in einem 2. Reaktionszyklus problemlos dem Reaktionsgemisch auf Stufe c) zugemischt werden. Da durch diese Tartrat-Fällung ein Teil der Calcium-Ionen aus dem Reaktionsgemisch entfernt wird, kann bei der anschließenden Calciumionen-Fällung mit einem Unterschuß an $Na_2CO_3$, bezogen auf in Stufe c) eingesetzte $Ca^{++}$-Ionen gearbeitet werden. Zur Entfernung dieser $Ca^{++}$-Ionen wird das Reaktionsgemisch bei 20-105 °C vorzugsweise 50-100 °C, mit festem $Na_2CO_3$ oder Natriumhydrogen-

carbonat, Mischungen davon oder deren Lösungen versetzt und 30 min bis 5 h, vorzugsweise 1-4 h, auf 70-105 °C erhitzt. Das dabei gebildete Calciumcarbonat läßt sich problemlos durch Filtration oder Abzentrifugieren abtrennen und in einem 2. Reaktionszyklus auf Stufe c)-als Katalysator einsetzen. Die Abtrennung des Calciumtartrats und -carbonats kann auch gleichzeitig durchgeführt werden.

Die erhaltene Reaktionslösung kann als solche verwendet oder aufkonzentriert werden. Auch ist es möglich, das Produkt durch Sprühtrocknung in Pulverform zu isolieren. Eine weitere Möglichkeit zur Entfernung der Calciumionen und zusätzlich der Natriumionen bietet die Verwendung eines Ionenaustauschers, mittels dessen die TMS/TDS-Salz-Lösung in die Form der freien Säuren übergeführt werden kann.

Zur Reinigung des Produktes kann sich gegebenenfalls eine wie in DE-AS 2 319 429 beschriebene Methanolfällung des TMS/TDS-Gemisches anschließen. Dabei wird eine Lösung der Natriumsalze von TMS/TDS in Wasser mit 0,5 bis 10 Gewichtsteilen Methanol pro Gewichtsteil wäßrige Lösung versetzt, wobei insbesondere die Natriumsalze von TMS und TDS gefällt werden und die Salze der Verunreinigungen in Lösung bleiben. Dieser Vorgang kann mehrmals wiederholt werden. Das isolierte Produkt wird z.B. im Trockenschrank getrocknet.

Durch das vorstehend beschriebene Verfahren gelingt es, ausgehend von Maleinsäure, in einer mehrstufigen Reaktionsfolge die Titelverbindungen herzustellen unter Verzicht auf den gesonderten Einsatz von teurer D,L-Weinsäure.

Beispiel 1

98 Teile Maleinsäureanhydrid werden bei 60 °C in 100 Teilen Wasser gelöst. Es werden 0,9 Teile $Na_2WO_4 \bullet 2H_2O$ zugegeben und anschließend 53 Teile 35 %ig. Wasserstoffperoxid bei 60-65 °C unter Kühlung in 10 min zugetropft. Es wird 3 h bei 70 °C und $1\frac{1}{2}$ h zum Rückfluß erhitzt.

Nach dem Abkühlen auf 60 °C werden 40 Teile Natronlauge und 40 Teile Calciumhydroxid zugegeben. Das Reaktionsgemisch wird mit konz. Natronlauge auf pH 12,0 eingestellt und 3 h auf 93-95 °C erhitzt. Während dieser Zeit entsteht aus der milchigen Suspension eine klare, homogene Lösung. Die Reaktion wird mittels HPLC verfolgt.

Nach dem Abkühlen auf 60 °C wird mit 100 Teilen Wasser verdünnt und mit konzentrierter Salzsäure auf pH 8 eingestellt und das gebildete Calciumtartrat abfiltriert. Zur vollständigen Calciumfällung werden 42 Teile Natriumcarbonat zum Filtrat gegeben und 1 h auf 80 °C erwärmt. Das gebildete Calciumcarbonat wird abfiltriert und mit wenig kaltem Wasser gewaschen.

Man erhält 449 g hellgelbe Lösung mit einem Feststoffgehalt von 32,5 % und folgender Zusammensetzung an organischen Komponenten:

3,8 % Weinsäure
1,5 % Apfelsäure
65,5 % TMS
7,4 % TDS
13,3 % Fumarsäure
8,4 % Maleinsäure

Beispiel 2

Es wird nach Beispiel 1 verfahren. Nach der Carbonat-Fällung werden in 346 Teile einer 22 %igen Lösung des Filtrats unter heftigem Rühren 600 Teile Methanol eingetragen. Es wird 1 h nachgerührt und anschließend vom Niederschlag abdekantiert. Der Niederschlag wird in 70 Teilen Wasser gelöst und das Produkt mit 300 Teilen Methanol gefällt. Der Niederschlag wird abfiltriert und mit 100 Teilen Methanol gewaschen. Das Produkt wird im Vakuumtrockenschrank 16 h bei 100 °C getrocknet. Man erhält 56,5 g weißes Pulver folgender Zusammensetzung an organischen Komponenten:

2,1 % meso-Weinsäure
4,2 % D,L-Weinsäure
82,0 % TMS

10,3 % TDS
0,1 % Fumarsäure
0,4 % Maleinsäure
1,0 % Apfelsäure

Beispiel 3

98 Teile Maleinsäureanhydrid werden bei 60 °C in 100 Teilen Wasser gelöst. Es werden 0,9 Teile $Na_2WO_4 \cdot 2H_2O$ zugegeben und anschließend 106 Teile 35 %ig. Wasserstoffperoxid innerhalb 15 min unter Kühlung bei 65-70 °C zugetropft. Es wird 3 h bei 70 °C und 1 h bei 100 °C nachgerührt. Nach dem Abkühlen auf 70 °C werden 88 Teile Maleinsäureanhydrid zugefügt und 15 min bei 70 °C nachgerührt. Anschließend werden 75 Teile Natronlauge und 75 Teile Calciumhydroxid zugefügt und der pH-Wert mit konz. Natronlauge auf pH = 11,9 eingestellt. Die milchige Suspension wird 3 h auf 95 °C erhitzt. Während dieser Zeit entsteht eine klare Lösung.

Durch Zugabe von Salzsäure wird der pH-Wert der Lösung auf 8,5 eingestellt und das Reaktionsgemisch 1 h auf 95 °C erhitzt. Nach dem Abkühlen wird das entstandene Calciumtartrat abfiltriert. Das Filtrat wird, wie in Beispiel 1 und 2 beschrieben, der Natriumcarbonat-Fällung und der 2-fachen Methanol-Fällung unterworfen. Der weiße Niederschlag hat folgende Zusammensetzung an organischen Komponenten:

1,8 % m-Weinsäure
2,4 % D,L-Weinsäure
0,3 % Apfelsäure
81,8 % TMS
13,0 % TDS
0,1 % Fumarsäure
0,6 % Maleinsäure

Beispiel 4

96 Teile Maleinsäureanhydrid werden bei 60 °C in 200 Teilen Wasser gelöst. Es werden 3,3 Teile $Na_2WO_4 \cdot 2H_2O$ hinzugegeben und anschließend unter Kühlung 81,6 Teile 50 %ig. $H_2O$ innerhalb 15 min zugetropft. Es wird 3 h bei 70 °C und 1½ h bei 100 °C nachgerührt. Nach dem Abkühlen auf 50 °C werden die Wolframionen durch Filtration über 100 Teile Ionenaustauscher des Typs IRA 400 entfernt.

Es werden 80 Teile Natriumhydroxid und 80 Teile Calciumhydroxid zugegeben und das Reaktionsgemisch bei 80 °C mit konzentrierter Natronlauge auf pH = 12,1 (gemessen in 10 %iger Lösung bei 25 °C) eingestellt und anschließend 3 h auf 95 ± 1 °C erhitzt. Während dieser Zeit entsteht aus der anfangs dickflüssigen Suspension eine klare homogene Lösung. Nach beendeter Reaktion wird das Reaktionsgemisch mit 100 Teilen Wasser verdünnt und durch Zugabe von D,L-Weinsäure auf pH = 8,5 eingestellt. Nach 1 stündigem Erhitzen auf 90 °C wird das Reaktionsgemisch auf 10 °C abgekühlt und das entstandene Calciumtartrat abzentrifugiert. Mittels Hochdruckflüssigkeitschromatographie wird die Zusammensetzung der organischen Komponenten wie folgt bestimmt.

2,8 % m-Weinsäure
3,6 % D,L-Weinsäure
1,4 % Apfelsäure
69,2 % TMS
9,9 % TDS
9,8 % Fumarsäure
3,3 % Maleinsäure

Beispiel 5

Analog Allan's Vorschrift (J. Catal. 19, 256-263 (1970); US 3,156,706) wird aus "Amberlite" IRA 400 und $Na_2WO_4 \bullet H_2O$ der polymere Epoxidationskatalysator hergestellt. 100 Teile dieses Katalysators werden zu einer Lösung von 116 Teilen Maleinsäure in 100 Teilen Wasser gegeben. Bei 70 °C werden 68 Teile 50 %ig. Wasserstoffperoxid (100 % Überschuß) unter Kühlung innerhalb 1 h zum Reaktionsgemisch getropft. Es wird 1 h bei 70 °C nachgerührt, anschließend 1 h unter Rückfluß erhitzt. Der polymer gebundene Katalysator wird abfiltriert und 37 Teile Calciumhydroxid-Pulver werden zur Reaktionslösung portionsweise innerhalb 10 min bei 80 °C zugegeben. Mit 70 %iger Natronlauge wird der pH-Wert der Lösung auf pH = 12,2 eingestellt und das Raktionsgemisch 3 h auf 93 °C erhitzt. Während dieser Zeit entsteht aus dem milchigen Reaktionsgemisch eine klare Lösung. Mit D,L-Weinsäure wird das Reaktionsgemisch auf pH = 8,5 eingestellt und 30 min auf 75 °C erhitzt. Das gebildete Calciumtartrat wird abfiltriert und das Filtrat zur vollständigen Fällung der Calciumionen mit 12 Teilen Natriumcarbonat 1 h auf 90 °C erhitzt. Nach dem Abkühlen werden 12 Teile Calciumcarbonat abfiltriert und mit wenig Wasser ausgewaschen. Das fast farblose Filtrat besitzt folgende Zusammensetzung an organischen Bestandteilen (bestimmt mittels HPLC):

6,2 % Weinsäure
66,3 % TMS
15,0 % TDS
4,0 % Fumarsäure
8,2 % Maleinsäure

## Ansprüche

1. Verfahren zur Herstellung von 1-Hydroxy-3-oxapentan-1,2,4,5-tetracarbonsäure und 3,6-Dioxa-octan-1,2,4,5,7,8-hexacarbonsäure, deren Salzen und deren Mischungen, dadurch gekennzeichnet, daß man ohne Zwischenisolierung

a) 1 Mol Maleinsäureanhydrid mit 0,2 bis 1,5 Mol Wasserstoffperoxid in Gegenwart von Epoxidations-Katalysatoren epoxidiert,

b) das dabei erhaltene Reaktionsgemisch durch Erhitzen in wäßrigem Medium hydrolysiert,

c) nach Zugabe von Erdalkali-oder Zinksalzen als Katalysator bei pH Werten von 10 bis 14 auf 60 bis 110°C erhitzt und dann

d) aus der so erhaltenen Lösung die oben genannten Endprodukte isoliert.

2. Verfahren nach Anspruch 1, dadurch gekennzeichnet, daß man in der Stufe a) 1 Mol Maleinsäure mit 0,8 bis 1,2 Mol $H_2O_2$ umsetzt und dann in den Stufen b) oder c) eine solche Menge an Maleinsäure zusetzt, daß das Mol-Verhältnis Weinsäure zu Maleinsäure in Stufe c) 1 : 0,5 bis 1 : 2,5 beträgt.

3. Verfahren nach Anspruch 1, dadurch gekennzeichnet, daß man in der Stufe d) die wäßrige Lösung der Endprodukte nach Abtrennen der Nebenprodukte sprühtrocknet.

4. Verwendung der nach Anspruch 1 hergestellten Verbindungen als Builder in Wasch-und Reinigungsmitteln.

Europäisches Patentamt

# EUROPÄISCHER RECHERCHENBERICHT

Nummer der Anmeldung

EP 87 11 6086

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | GB-A-2 183 633 (THE PROCTER & GAMBLE) * Seite 2, Zeile 4 - Seite 10, Zeile 15 * | 1,2,4 | C 07 C 59/305<br>C 07 C 51/00<br>C 11 D 3/20 |
| A | DE-A-2 408 591 (HENKEL) * Gesamt * | 1,4 | |
| D,A | US-A-3 914 297 (V. LAMBERTI et al.) * Zusammenfassung; Ansprüche * | 1,4 | |
| P,X | US-A-4 663 071 (R.D. BUSH et al.) * Spalte 3, Zeilen 3-34; Spalte 5, Zeile 27 - Spalte 8, Zeile 64; Beispiel 1; Anspruch 1 * | 1,3,4 | |
| | | | **RECHERCHIERTE SACHGEBIETE (Int. Cl.4)** |
| | | | C 07 C 59/00<br>C 11 D |

Der vorliegende Recherchenbericht wurde für alle Patentansprüche erstellt

| Recherchenort | Abschlußdatum der Recherche | Prüfer |
|---|---|---|
| WIEN | 07-01-1988 | HOFBAUER |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)